# EUROPEAN PATENT APPLICATION

(11) **EP 1 653 225 A1**
(43) Date of publication of application: **03.05.2006**
(21) Application number: 04425852.3
(22) Date of filing: 15.11.2004
(51) Int. Cl.: G01N 21/89, G01N 33/36

(54) **Device for detecting flaws and/or foreign material in yarn or textile web**

(30) Priority: 26.10.2004 WO PCT/IT20/04000584
(71) Applicant: Franzoni Filati S.p.A., 25040 Esine (Brescia) (IT)
(72) Inventor: Olmi, Fausto, Franzoni Filati S.p.A., 25040 Esine (Brescia) (IT); Fiorini, Armando, Franzoni Filati S.p.A., 25040 Esine (Brescia) (IT)
(74) Representative: Crippa, Paolo Ernesto

(57) **Abstract**

A device for detecting flaws and/or impurities in a product (1), such as a yarn made of textile materials, comprising a detection region (2) crossed by the yarn at a set running speed, at least one laser emitter (4) and at least one photodiode (24) for receiving a ray (20) reflected by the yarn. The wavelength of the emitted ray is chosen according to the running speed and/or the characteristics of the yarn to be analysed.

## Description

The present invention relates to a device for detecting flaws and/or impurities in a product during the manufacturing cycle of the product.

In particular, a device for detecting flaws and/or impurities in a yarn of textile materials, for example cotton, or a thin web of said material, constitutes the subject of the present invention.

Being able to make a quick check of the product obtained to detect the presence of flaws or impurities is a well-known requirement in all sectors of production, in which a product is made with a particularly prevalent extension in one direction, for example in the sense of the length, such as a yarn of textile materials, plastic materials, metal materials, a thin web of textile materials and the like.

Understandably, various problems are encountered when satisfying this requirement.

In fact the check must be reliable to allow parts of the product to be identified and, if necessary, discarded, when they do not satisfy the standard requirements of the required production.

At the same time, the check must be quick to adapt to the manufacturing speeds and be carried out during the course of the product manufacturing cycle.

Moreover, the means used to carry out this check must be economic to allow a replacement that is not expensive during maintenance.

These requirements and the relative problems encountered in satisfying them are accentuated in the textile industry, and even more so particularly in the sector of producing yarn on reels.

Devices are known, called "slubcatchers" in slang in the sector, which are designed to detect flaws and/or impurities in a yarn during the manufacturing cycle of the yarn on reels.

Examples of embodiment of these devices are shown in the documents W02004/044579, US6,771,365 and CH693677.

However, these devices also present the aforesaid disadvantages.

It is the object of the present invention to realise a device to detect flaws and/or impurities to overcome the disadvantages stated with reference to the prior art.

**.** The features and advantages of the device according to the present invention will be recognised from the following description, given by way of example, which is not limiting, with reference to the textile industry and with reference to detecting impurities and/or flaws in a yarn of textile materials, for example cotton.

**.** The following description is given with reference to the accompanying figures, wherein:

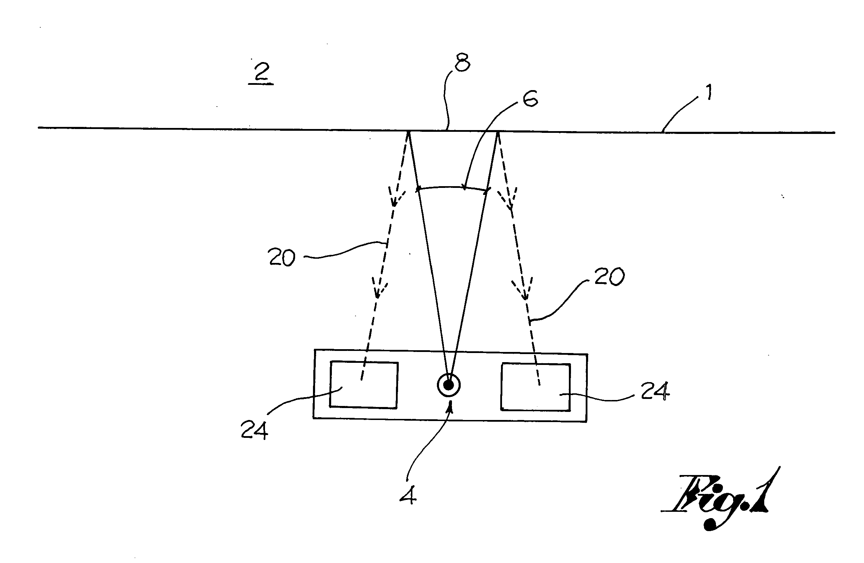

**.** - figure 1 shows a scheme of a detection unit of the device according to the present invention;

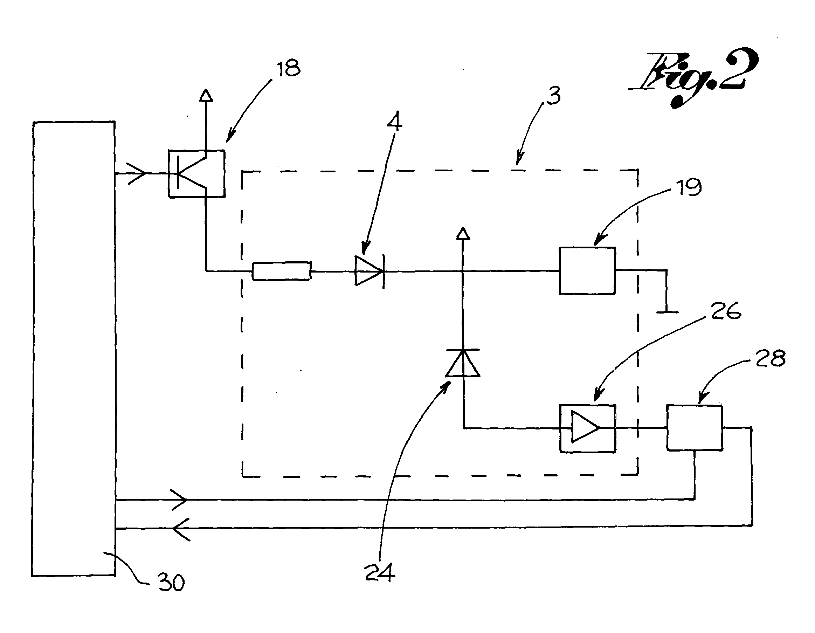

**.** - figure 2 represents a structural and functional scheme of the detection unit in figure 1, connected to accessory means of the device 1;

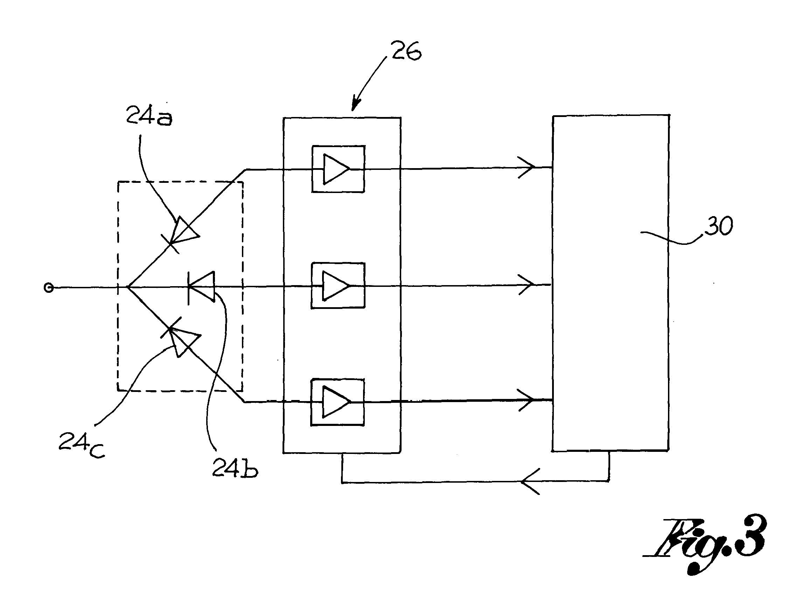

**.** - figure 3 shows a structural and functional scheme of a detection unit according to the present invention suitable for detecting flaws, such as chromatic variations of the product;

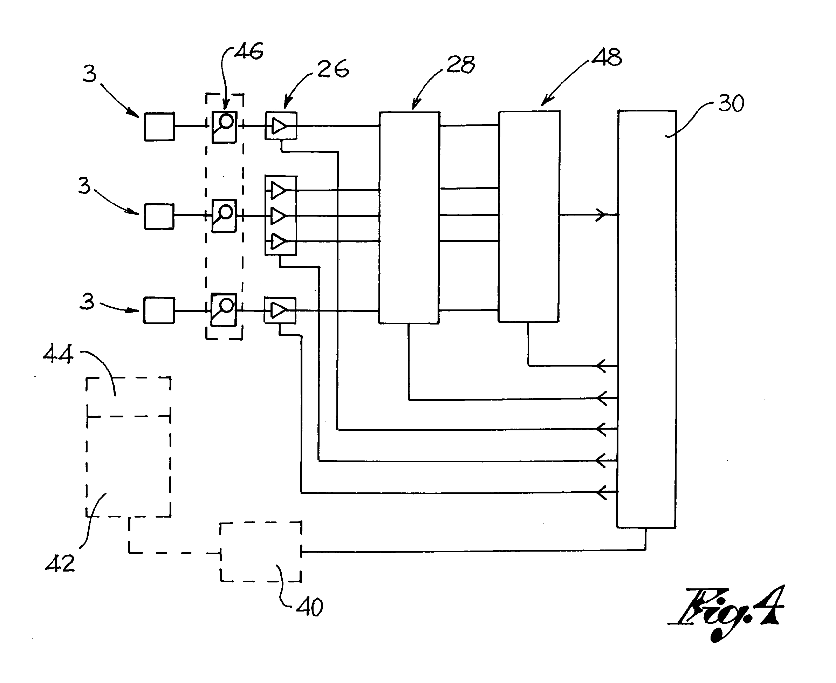

**.** - figure 4 represents a structural and functional scheme of the device according to the present invention, according to an embodiment, connected to a machine;

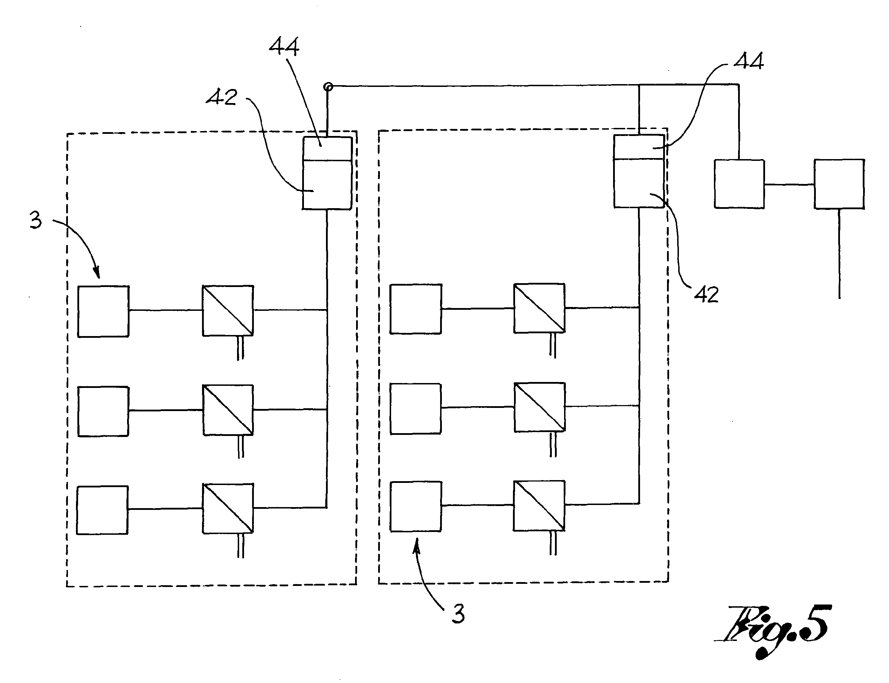

**.** - figure 5 shows a structural and functional scheme of a system for managing a plurality of machines connected to the device according to the present invention.

**.** According to a preferred embodiment, the device, according to the present invention, comprises a box suitable for being connected to a machine introduced into a system for textile working.

**.** For example, said machine is a spooler, in other words a machine designed to wind a yarn 1 on to reels for subsequent working.

**.** Advantageously, the detection device is positioned upstream of the reels. During the course of the working, the yarn 1, which runs at a set speed, crosses said box to carry out said detecting of the flaws and/or impurities and is then wound onto the reels.

**.** Inside said box, the device preferably comprises a detection region 2 through which the yarn 1 runs; said running is realised at a set running speed.

**.** Additionally, the device comprises a detection unit 3, comprising emitter means 4 suitable for emitting a ray emitted 6 towards said detection region 2.

**.** The emitted ray 6 influences a part 8 of the yarn 1 to be analysed.

**.** Said emitter means 4 are suitable for emitting an emitted ray 6 basically with a single emission wavelength, said wavelength being chosen according to said set running speed and said yarn to be analysed.

**.** In particular, said emitter means comprise a laser emitter, whose emission characteristics, in particular the wavelength of the ray emitted, are chosen according to said set running speed and said yarn to be analysed.

**.** According to a preferred embodiment, said laser emitter presents a wavelength of the ray that is emitted of between 750 and 950 nanometres inclusive, preferably between 800 and 900 nanometres, preferably between 820 and 860 nanometres.

**.** It is understood in the sphere of the present invention that stating that said emitter means emit at a single wavelength means that the ray emitted has an extremely limited wavelength band, typical of the constructional characteristics of lasers.

**.** According to a further embodiment of the detection unit, said emitter means comprise means for focusing the ray that is emitted.

**.** Said means for focusing the ray that is emitted comprise at least one lens set along the path of the emitted ray, suitable for limiting the opening angle of the emission cone of said laser emitter.

**.** Said lens is preferably suitable for limiting said opening angle at a width of between 15 hexagesimal degrees and 30 hexagesimal degrees inclusive, preferably between 18 hexagesimal degrees and 25 hexagesimal degrees. Said opening angle is preferably equal to 20 hexagesimal degrees.

**.** According to a preferred embodiment, said detection unit comprises stabilising means suitable for stabilising the ray emitted, in other words suitable for keeping the characteristics of said ray emitted constant in time.

**.** In particular, said stabilising means are suitable for preventing the influence of external disturbances, such as electrostatic charges for example on said laser emitter.

**.** Advantageously, said stabilising means are suitable for limiting or cancelling the negative effects that the electrostatic charges lying on the yarn can have on the laser emitter.

**.** According to a preferred embodiment, said stabilising means comprise electronic devices suitable for fixing a voltage supply that is basically constant in at least two points over said laser emitter.

**.** In other words, said electronic devices are suitable for fixing a first supply voltage, for example equal to 7 Volts, upstream of the laser emitter, and a second voltage, for example equal to 5 Volts, downstream of said laser emitter.

**.** According to a preferred embodiment, said electronic devices comprise an optoisolator 18 that is operatively connected upstream of the laser emitter. According to an even further preferred embodiment, said electronic devices comprise a supply filter 19 that is operatively connected downstream of said laser emitter.

**.** According to a preferred embodiment, said device comprises modulation devices suitable for allowing the frequency modulation of the emission of said emitted ray.

**.** If activated, said modulation devices allow said emitted ray to be emitted at a set emission frequency.

**.** Moreover, said detection unit comprises receptor means suitable for receiving a ray that is reflected 20 from said part of the yarn 1 to be analysed, said reflected ray 20 being basically made up of the reflection on said part to be analysed of said emitted ray.

**.** In other words, said emitted ray influences a part of the running yarn. Said part affected by the emitted ray forms the part of the yarn 1 to be analysed.

**.** Following the effect of the emitted ray on the part to be analysed, there is a partial or total reflection of said ray, thus creating a reflected ray that is intercepted by said receptor means.

**.** According to a preferred embodiment, said receptor means comprise at least one photodiode 24 suitable for being sensitised by said reflected ray.

**.** Said photodiode is chosen according to the wavelength characteristic of the ray emitted by the emitter means.

**.** Said photodiode is preferably made of silicon, complete with an anti-reflection cover and operation in photovoltaic or photoconductive mode, suitable for working on a broad band.

**.** According to a preferred embodiment, said receptor means comprise a pair of photodiodes.

**.** According to a further embodiment, said receptor means comprise a vector of photodiodes, or a plurality of photodiodes arranged along a set direction. According to an even further embodiment, said receptor means comprise a matrix of photodiodes, or a plurality of photodiodes arranged according to two set directions to form a reception matrix.

**.** According to a preferred embodiment, said receptor means are suitable for detecting colouring flaws in the yarn.

**.** According to this embodiment, said receptor means comprise three photodiodes 24a, 24b, 24c, which are sensitive to red, green and blue respectively.

**.** According to a preferred embodiment, said modulation means are suitable for rejecting the signals acquired by said receptor means with a frequency that is different from the emission frequency determined by said modulation means.

**.** Advantageously, this allows the signals created by unwanted disturbances to be effectively rejected or isolated.

**.** Moreover, according to a preferred embodiment, said detection unit 3 comprises at least one amplification unit 26 operatively connected to said receptor means. In particular, said amplification unit 26 is operatively connected to said photodiode 24.

**.** Moreover, according to a preferred embodiment, said device comprises a signal analysis unit 28 that can be connected to said detection unit 3, in particular that can be operatively connected to said amplification unit 26.

**.** Moreover, according to a preferred embodiment, said device comprises a data processing unit 30 (CPU) that can be connected to said signal analysis unit 28. Moreover, said data processing unit 30 is operatively connected to said optoisolator 18.

**.** Said detection device preferably comprises a plurality of detection units. For example, said device comprises detection units suitable for detecting the presence of impurities and detection units suitable for detecting the presence of flaws.

**.** Said data management unit can also be connected to a module for managing the input and output signals 40, to a centralised module 42 and to a machine management unit 44.

**.** Said device preferably comprises at least one optical amplifier 46 that is operatively connected to said detection unit 3 and to said signal analysis unit 28.

**.** Said device also preferably comprises a programmable analogical multiplexer 48 that is operatively connected to said data processing unit 30 and to said signal analysis unit 28.

**.** According to a preferred form of system management, a plurality of management units of a machine are operatively connected to a general centralised module, which is connected, in turn, to a remote control unit, for example to a personal computer.

**.** A set quality of yarn for production is identified by a set importance of the flaws, which can be found in it, for example chromatic variations, variations in diameter and the like, and a set importance of the impurities present, for example fibres of unrelated material, generally not vegetable, for example polypropylene fibres, manufacturing residues and the like.

**.** To produce a set quality of yarn, detection is carried out using the device according to the present invention on a yarn with the desired quality called a sample-yarn.

**.** The sample-yarn is analysed by the aforesaid device.

. The ray emitted by the laser emitter influences the sample-yarn parts, which run across the detection region. The emitted ray is characterised by presenting a basically single wavelength, typical of the laser emitter used.

. Part of the emitted ray, influencing the part of the yarn to be analysed, is dispersed according to various directions inside the detection area 2. Whereas part of said ray is reflected, forming the reflected ray 20.

. The ray that is reflected by the sample-yarn is received by the receptor means, which are basically arranged on the same side as the laser emitter in relation to the yarn.

. The reflected ray presents a plurality of wavelengths because of the reflection characteristics of the sample-yarn and the running speed.

. The signal processing means process the signal from the receptor means, organising these signals into a sample-spectrum, in other words, for example into a diagram in which, the intensity of the wavelengths is reported according to the wavelengths, which are detected.

. Said sample-spectrum is memorised by said signal processing means.

. During the use of the device according to the present invention, the yarn being produced crosses said detection region at a set running speed.

**.** The ray that is emitted by the laser emitter influences the parts of the yarn, which run across the detection region. The ray that is emitted is characterised by presenting a single wavelength or emission frequency, typical of the laser emitter used.

**.** The ray that is reflected by the yarn is received by the receptor means. The reflected ray presents a plurality of wavelengths because of the reflection characteristics of the yarn and the running speed.

**.** The signal analysis unit processes the signal from the receptor means, organising these signals into a spectrum.

**.** Said spectrum is compared with the previously memorised sample-spectrum.

**.** The differences between said sample-spectrum and said production spectrum enable flaws and/or impurities in the yarn being produced to be detected.

**.** Innovatively, the device according to the present invention allows flaws and/or impurities to be detected in a product that runs in the detection region at high speed.

**.** Typical running speeds, for example in the textile industry, are about 2000 m/min (=33 m/sec approx.).

**.** Advantageously, the laser emitter enables a ray to be used that is emitted with a single set wavelength, which is suitable for the reflection on the yarn being produced and for the running speed.

**.** Also advantageously, depending on the typology of yarn being produced, various detection units can be activated and/or deactivated to detect, for example, only the flaws or only the impurities in the yarn being produced.

**.** Note that the term 'impurity' usually indicates the presence of dirt and/or fibres of material that are unrelated to the fibre, for example fibres of polypropylene and the like. Whereas the term 'flaws' usually indicates an unwanted chromatic variation along the length of the yarn and/or knots or curling of the fibre and the like.

**.** Clearly an expert in the sector can make several variations and modifications to the above described device to satisfy specific contingent needs, all of which are also included in the scope of protection, as defined by the following claims.

## Claims

1. Device for detecting flaws and/or impurities in a product (1), wherein said product presents a prevalent longitudinal extension in relation to one of its transversal extensions, such as a yarn, a ribbon, a thin web and the like, wherein said device comprises
- a detection region (2) suitable for being crossed at a set running speed to detect flaws and/or impurities;
- emitter means (4) suitable for emitting a ray (6) emitted towards said detection region, said emitted ray influencing a part of said product (1) to be analysed (8);
- receptor means suitable for being sensitised by a ray that is reflected (20) from said part of the product (1) to be analysed (8), said reflected ray being basically made up of the reflection on said part to be analysed of said emitted ray; said device being **characterised in that** said emitter means are suitable for emitting a ray that is emitted basically with a single wavelength, said wavelength being chosen according to said set running speed and/or said product to be analysed.

2. Device according to claim 1, wherein said emitter means comprise a laser emitter.

3. Device according to claim 2, wherein said emitter means comprise a laser emitter suitable for emitting a ray with a wavelength of between 820 and 860 nanometres inclusive.

4. Device according to any one of the previous claims, comprising modulation means suitable for allowing the emission of said ray that is emitted with a set emission frequency.

5. Device according to any one of the previous claims, wherein said receptor means comprise at least one photodiode (24).

6. Device according to claim 5, wherein said receptor means comprise a vector of photodiodes.

7. Device according to claim 5, wherein said receptor means a matrix of photodiodes.

8. Device according to any one of the previous claims, wherein said receptor means comprise a photodiode sensitive to red, a photodiode sensitive to green and a photodiode sensitive to blue.

9. Device according to any one of the previous claims, wherein said device comprises stabilising means for said emitter means.

10. Device according to claim 9, wherein said stabilising means comprise electronic devices suitable for fixing a first supply voltage at a basically constant value upstream of a laser emitter and a second voltage that is basically constant downstream of said laser emitter.

11. Device according to any one of the previous claims, also comprising a signal analysis unit (28) that is operatively connected to said receptor means.

12. Device according to any one of the previous claims comprising memorisation means suitable for memorising a sample-spectrum of an analysed sample-product.

13. Device according to claim 12 also comprising comparing means suitable for comparing a production spectrum detected on a production product with a sample-spectrum of a sample-product.

14. Device according to claim 13, wherein said memorisation means are operatively connected to said comparing means.

15. Device according to any one of the previous claims, wherein said emitter means comprise focusing means of the emitted ray, which are suitable for limiting the opening angle of the emission cone of said emitted ray.

16. Device according to claim 15, wherein said focusing means of the emitted ray comprise at least one lens set along the path of the ray that is emitted.

17. Device according to claim 15 or 16, wherein said focusing means are suitable for limiting said opening angle at a width of between 15 hexagesimal degrees and 30 hexagesimal degrees inclusive.

18. Device according to claim 17, wherein said focusing means are suitable for limiting said opening angle at a value of between 18 hexagesimal degrees and 25 hexagesimal degrees inclusive.

19. Device according to claim 18, wherein said opening angle is basically equal to 20 hexagesimal degrees.
